# EUROPÄISCHE PATENTSCHRIFT

(11) **EP 2 645 998 B1**
(45) Veröffentlichungstag und Bekanntmachung des Hinweises auf die Patenterteilung: **20.08.2014**
(21) Anmeldenummer: 11794675.6
(22) Anmeldetag: 30.11.2011
(51) Int. Cl.: A61K 38/02, A61K 38/36, A61K 9/14, A61K 31/728, A61K 38/19, A61K 9/00, A61K 9/16

(54) **VERWENDUNG VON ZYTOKINE-FREISETZENDEN, BIODEGRADIERBAREN PARTIKELN IN HYALURONSÄURE ZUR BEHANDLUNG VON KNORPELDEFEKTEN, INSBESONDERE VON OSTEOARTHROSE**
USE OF CYTOKINE-RELEASING, BIODEGRADABLE PARTICLES IN HYALURONIC ACID FOR THE TREATMENT OF CARTILAGE DEFECTS, IN PARTICULAR OF OSTEOARTHROSIS
UTILISATION DE PARTICULES BIODÉGRADABLES LIBÉRANT DES CYTOKINES DANS DE L'ACIDE HYALURONIQUE POUR LE TRAITEMENT DE LÉSIONS DU CARTILAGE, EN PARTICULIER DE L'ARTHROSE

(30) Priorität: 01.12.2010 DE 102010062288
(43) Veröffentlichungstag der Anmeldung: 09.10.2013
(73) Patentinhaber: Charité Universitätsmedizin Berlin, 10117 Berlin (DE)
(72) Erfinder: SITTINGER, Michael, 12305 Berlin (DE); RINGE, Jochen, 16540 Hohen Neuendorf (DE); ANDREAS, Kristin, 10409 Berlin (DE)
(74) Vertreter: Gulde & Partner
(86) Internationale Anmeldenummer: PCT/EP2011/071412
(87) Internationale Veröffentlichungsnummer: WO 2012/072692

(56) Entgegenhaltungen:
- WO-A1-93/07861
- WO-A1-2005/014027
- WO-A1-2010/123900
- WO-A2-01/28591
- US-A1- 2010 285 113
- US-B1- 6 428 804
- HEGEWALD A A ET AL: "Hyaluronic acid and autologous synovial fluid induce chondrogenic differentiation of equine mesenchymal stem cells: a preliminary study", TISSUE AND CELL, CHURCHILL LIVINGSTONE MEDICAL JOURNALS, EDINBURGH, GB, Bd. 36, Nr. 6, 1. Dezember 2004 (2004-12-01), Seiten 431-438, XP004631329, ISSN: 0040-8166, DOI: 10.1016/J.TICE.2004.07.003

## Beschreibung

Laut dem Statistischen Bundesamt gehört die Osteoarthrose (OA, Gelenkverschleiß), insbesondere die Osteoarthrose der Knie- und Hüftgelenke, zu den 30 häufigsten Einzeldiagnosen bei stationären Krankenhausaufenthalten. Im Jahr 2007 waren ca. 15 Millionen Arthrosepatienten in Behandlung. Mit der in Deutschland zu erwartenden demographischen Entwicklung ist in der Zukunft mit einem weiter wachsenden Versorgungsbedarf zu rechnen.

Trotz einiger Behandlungsmöglichkeiten gibt es bis heute keine Therapieform, mit der sich der Krankheitsprozess der Osteoarthrose tatsächlich aufhalten oder sogar umkehren lässt. Für kleine, lokal begrenzte Knorpeldefekte wurden Therapien entwickelt, wie beispielsweise die autologe Chondrozytentransplantation oder die Implantation zellfreier Implantate. Allerdings können diese Therapien eine weitere Knorpeldegeneration nicht aufhalten und eignen sich zudem nicht für den Einsatz bei großflächigeren OA-Defekten. Meist führt die Osteoarthrose früher oder später zum Gelenkersatz. Pro Jahr werden ca. 420 000 Arthrose-Operationen durchgeführt, bei denen eine Totalendoprothese implantiert wird. Die erforderliche ambulante und stationäre Versorgung verursacht sehr hohe Kosten. Nach Berechnungen des statistischen Bundesamtes wurden im Jahr 2002 für die Behandlung von Arthrosen ca. 7 Milliarden Euro aufgewendet. Darüber hinaus führt diese chronischdegenerative Erkrankung zusätzlich zu einem erheblichen volkswirtschaftlichen Aufwand infolge von Berufsunfähigkeit, Frühberentung und Rehabilitationsmaßnahmen von betroffenen Menschen.

Für fortgeschrittene Osteoarthrose-Defekte gibt es neben der Totalendoprothese und einer Schmerztherapie weitere Behandlungskonzepte, wie beispielsweise die Applikation von Glucosamin/Chondroitin und Ernährungstherapien. Die Wirksamkeit solcher Therapien ist bislang allerdings kaum ausreichend belegt. Lediglich die intra-artikuläre Applikation von Hyaluronsäure (HS oder auch mit HA abgekürzt), die als zusätzliche "Gelenkschmiere" fungieren soll, zeigte in prospektiv kontrollierten Studien einen positiven Effekt. Eine weitere Knorpeldegeneration konnte verzögert werden. Eine Knorpelregeneration konnte nicht belegt werden.

WO 2005/014027 offenbart Zusammensetzungen enthaltend Hyaluronsäure und CCL 19.

Es bleibt ein Bedarf an weiteren effektiven Therapieformen zur Behandlung der Osteoarthrose, die ein Aufhalten der Knorpeldegeneration und ggf. sogar eine Regeneration von erkranktem Knorpelgewebe erlaubt und damit die Implantation einer Totalendoprothese verzögert oder sogar überflüssig macht.

Aufgabe der vorliegenden Erfindung ist es einen oder mehrere Nachteile des Standes der Technik zu vermindern oder zu vermeiden. Insbesondere ist es eine Aufgabe der Erfindung neue effektive Mittel und Wege zur Therapie von Osteoarthrose bereitzustellen.

Die vorliegende Erfindung löst diese Aufgabe durch Bereitstellung einer Zusammensetzung, die

| | |
|---|---|
| 2 - 50 mg/ml | Hyaluronsäure, |
| 0,1 - 500 mg/ml | biodegradierbare Partikel mit einem durchschnittlichen mittleren Partikeldurchmesser von 1 nm - 500 µm, |
| 1 pg/ml - 10 µg/ml | Zytokine gemäß Anspruch 1, |

enthält oder daraus besteht, wobei sich die Konzentrationsangaben jeweils auf das Gesamtvolumen (w/v) der Zusammensetzung beziehen und wobei die Zytokine in den biodegradierbaren Partikeln eingeschlossen sind.

Die Erfindung beruht auf der überraschenden Erkenntnis, dass eine simultane Verabreichung von Hyaluronsäure und von Zytokinen in synergistischer Weise die Migration von am Aufbau eines gesunden Gelenks beteiligten Zellen stimuliert, wie beispielsweise mesenchymalen Stamm- und/oder Progenitorzellen. Dadurch wird in besonders vorteilhafter Weise die Rekrutierung von gesunden Zellen zur Geweberegeneration in Gelenken mit Knorpeldefekten, beispielsweise in von mit Osteoarthrose befallenen Gelenken oder in Gelenken mit traumatischen Knorpeldefekten, gefördert und verstärkt. Die positive Wirkung wird unter anderem dadurch erreicht, dass die Zytokine derart formuliert vorliegen, so dass diese nicht auf einmal freigesetzt werden, sondern kontrolliert über einen längeren Zeitraum. Dies wird in der erfindungsgemäßen Zusammensetzung dadurch erreicht, dass die Zytokine in Partikeln eingeschlossen vorliegen, wobei diese Partikel biodegradierbar sind. Nach der Applikation im Körper des zu behandelnden Patienten werden die Partikel über die Zeit abgebaut und setzen dabei kontinuierlich Zytokine frei.

Die Verabreichung der erfindungsgemäßen Zusammensetzung, insbesondere der gelartigen Hyaluronsäure in Kombination mit den Zytokin freisetzenden, biodegradierbaren Partikeln, in den Gelenkinnenraum ermöglicht eine praktische und viel versprechende Erweiterung der bisherigen Behandlungsmöglichkeiten von Knorpeldefekten, z.B. von traumatischen Knorpeldefekten oder Osteoarthrose. Neben der Verzögerung der Knorpeldegeneration durch die Hyaluronsäure, können die Zytokine-freisetzenden Partikel im Gelenksinnenraum zeitgleich und zusätzlich einen regenerativen Effekt auf den beschädigten Knorpel ausüben.

Nach einer intra-artikulären Verabreichung der erfindungsgemäßen Zusammensetzung verzögert die Hyaluronsäure, in seiner Eigenschaft als "Gelenkschmiere" und zusätzlich als chondrogener Faktor, eine weitere Degeneration des Knorpels und fördert die Knorpelneubildung. Die gleichzeitige, kontrollierte und über mehrere Tage oder sogar Wochen anhaltende, lokale Freisetzung von Zytokinen stimuliert gezielt Stamm- und/oder Progenitorzellen und/oder lockt diese an, so dass sich diese Zelltypen im Bereich der Osteoarthrose bzw. im erkrankten Bereich des Gelenks anreichern. Dabei wird insbesondere ausgenutzt, dass die Hyaluronsäure vermittelte Knorpelneubildung für die Rekrutierung und Ansiedlung von Stamm- und/oder Progenitorzellen eine unterstützende Rolle spielt. Weiterhin konnte gezeigt werden, dass die kombinierte Verabreichung von Hyaluronsäure und Zytokinen eine synergistische Wirkung auf die Rekrutierung von Stamm- und/oder Progenitorzellen hat. So wird durch die Injektion der erfindungsgemäßen Zusammensetzung eine gezielte Anreicherung von körpereigenen Stamm- und/oder Progenitorzellen in dem erkrankten Gelenk erreicht, während durch die Kombination mit der simultanen, lokalen Hyaluronsäure Gabe gleichzeitig ein Fortschreiten der Knorpeldegeneration verzögert als auch eine Regeneration des beschädigten Knorpelgewebes stimuliert wird.

Die erfindungsgemäße Zusammensetzung enthält Hyaluronsäure in einer Konzentration von 2 - 50 mg/ml bezogen auf das Gesamtvolumen (w/v) der Zusammensetzung. Bevorzugt enthält die erfindungsgemäße Zusammensetzung 5 - 30 mg/ml Hyaluronsäure, besonders bevorzugt 10 - 20 mg/ml, ganz besonders bevorzugt 10 oder 20 mg/ml, jeweils bezogen auf das Gesamtvolumen (w/v) der Zusammensetzung.

Verfahren zur Gewinnung bzw. Herstellung von Hyaluronsäure sind dem Fachmann bekannt. Hyaluronsäure wird üblicherweise aus Geweben extrahiert oder biotechnologisch, beispielsweise durch Fermentation, hergestellt.

Die Halbwertszeit von Hyaluronsäure nach der Applikation in Gewebe ist abhängig von der molaren Masse der Hyaluronsäure. Um eine besonders vorteilhafte Halbwertszeit der Hyaluronsäure nach Applikation der erfindungsgemäßen Zusammensetzung zu gewährleisten, enthält die erfindungsgemäße Zusammensetzung Hyaluronsäure mit einer durchschnittlichen mittleren molaren Masse von mindestens 200 kDa, bevorzugt von 250 kDa bis 7000 kDa, besonders bevorzugt von 500 kDa bis 4000 kDa, ganz besonders bevorzugt von 1000 kDa bis 3000 kDa.

Die erfindungsgemäße Zusammensetzung enthält biodegradierbare Partikel, in denen die Zytokine eingeschlossen vorliegen. Dabei weist die erfindungsgemäße Zusammensetzung die Partikel in einer Konzentration von 0,1 mg/ml bis 500 mg/ml auf, bevorzugt in einer Konzentration von 1 mg/ml bis 300 mg/ml, besonders bevorzugt von 5 mg/ml bis 200 mg/ml, jeweils bezogen auf das Gesamtvolumen (w/v) der Zusammensetzung. Eine erfindungsgemäße Zusammensetzung, die die biodegradierbaren Partikel in einer Konzentration von 50 mg/ml enthält, eignet sich besonders gut als Injektionslösung für die intra-artikuläre Injektion in zu behandelnde Gelenke. Dagegen eignet sich eine erfindungsgemäße Zusammensetzung, die die biodegradierbaren Partikel in einer Konzentration von 200 mg/ml enthält, besonders gut zur Herstellung einer Paste oder eines Gels für die arthroskopische Behandlung.

Die biodegradierbaren Partikel weisen einen durchschnittlichen mittleren Partikeldurchmesser auf von 1 nm bis 500 µm, bevorzugt von 10 nm bis 200 µm, besonders bevorzugt von 100 nm - 50 µm.

Unter dem Begriff "biodegradierbar" werden Partikel verstanden, die nach Implantation in einen menschlichen oder tierischen Körper oder Gewebe über die Zeit abgebaut und schließlich resorbiert und/oder ausgeschieden werden. Dazu können die Partikel der erfindungsgemäßen Zusammensetzung aus einem biokompatiblen, biodegradierbaren Polymer bestehen oder dieses enthalten. Unter Biokompatibilität oder Körperverträglichkeit wird die Fähigkeit eines Polymers verstanden, in einer spezifischen Anwendung eine angemessene Gewebereaktion hervorzurufen. Mit anderen Worten, ist ein Polymer dann biokompatibel, wenn das Polymer nach der Implantation im Körper oder Gewebe keine untolerierbaren unerwünschten Reaktionen hervorruft.

Als biodegradierbar im Sinne der Erfindung werden Polymere oder Partikel bezeichnet, bei denen in physiologischer Umgebung über die Zeit ein Abbau/Umbau stattfindet, so dass das Polymer oder der Partikel nach Implantation ganz oder zumindest überwiegend nicht mehr vorhanden ist. Als Prüfmedium zur Testung des Degradationsverhaltens von in Frage kommenden Polymeren oder Partikeln dient künstliches Plasma, wie es nach EN ISO 10993-15:2000 für Biodegradationsuntersuchungen vorgeschrieben ist (Zusammensetzung NaCl 6,8 g/l, CaCl₂ 0,2 g/l, KCl 0,4 g/l, MgSO₄ 0,1 g/l, NaHCO₃ 2,2 g/l, Na₂HPO₄ 0,126 g/l, NaH₂PO₄ 0,026 g/l). Das künstliche Plasma nach EN ISO 10993-15:2000 entspricht einem blutähnlichen Medium und stellt damit eine Möglichkeit dar, eine physiologische Umgebung im Sinne der Erfindung reproduzierbar nachzustellen. Eine Probe des zu untersuchenden Polymers oder Partikels wird dazu in einem verschlossenen Probenbehälter mit einer definierten Menge des Prüfmediums bei 37 °C gelagert. In zeitlichen Abständen - abgestimmt auf das zu erwartende Degradationsverhalten - von wenigen Stunden bis zu mehreren Monaten werden die Proben entnommen und in bekannter Weise auf Degradationsspuren untersucht.

Geeignete biokompatible und biodegradierbare Polymere umfassen Polymere oder CoPolymere, die ein Milchsäure- (LA-) und/oder ein Glykolsäure- (GA-) Monomer enthalten oder daraus bestehen, Poly-Milchsäure (PLA), Poly-Glykolsäure (PGA), Polylactid-co-Glycolid (PLGA), Polyethylenglycol (PEG), PLGA-PEG, Polycaprolactone, Polycarbonate, Polyamide, Polyanhydride, Chitosane, Dextrane, Cyclodextrine und/oder Fibrinogen. Besonders bevorzugt enthalten oder bestehen die biodegradierbaren Partikeln aus PLGA, wobei das PLGA-Polymer endgruppenmodifiziert sein kann. Das PLGA kann in D-, L- oder D-/L-Konfiguration verwendet werden. Bevorzugt weist das PLGA eine molare Masse von 5 kDa bis 100 kDa auf, besonders bevorzugt von 15 kDa.

Eingeschlossen in den biodegradierbaren Partikeln enthält die erfindungsgemäße Zusammensetzung 1 pg/ml bis 10 µg/ml Zytokine, bevorzugt 10 pg/ml bis 1 µg/ml Zytokine, besonders bevorzugt 100 pg/ml bis 100 ng/ml Zytokine, jeweils bezogen auf das Gesamtvolumen (w/v) der Zusammensetzung. Unter Zytokinen werden Proteine verstanden, die eine regulierende Funktion auf das Wachstum, die Migration und/oder die Differenzierung von Zellen ausüben. Zytokine umfassen sowohl Wachstumsfaktoren als auch Chemokine und Differenzierungsfaktoren. Erfindungsgemäß können insbesondere Zytokine eingesetzt werden, die mesenchymale Stamm- und/oder Progenitorzellen stimulieren und/oder aktivieren. Verwendet werden die Zytokine CXCL-10, CXCL-12, CCL25 und/oder XCL-1 bzw. eine Mischung enthaltend eines, mehrere oder alle vier dieser Zytokine. Insbesondere können die Zytokine CXCL-12 und/oder CCL-25 in der erfindungsgemäßen Zusammensetzung verwendet werden.

Die biodegradierbaren, Zytokin-tragenden Partikel können in an sich bekannter Art und Weise hergestellt werden. Geeignete Herstellverfahren ergeben sich aus der Wahl der jeweiligen Materialien aus denen die Partikel zusammengesetzt sind, insbesondere aus der Wahl der biokompatiblen, biodegradierbaren Polymeren. Übliche Herstellverfahren umfassen die Herstellung mittels Formulierung in Emulsion (z.B. w/o/w-, s/o/w- oder o/w-Emulsion), wobei meist das Zytokin in der wässrigen Phase gelöst vorliegt und in einer organischen Polymerphase fein verteilt wird und anschließender Lösungsmittelverdunstung, die Herstellung mittels Phasenseparation, Sprühtrocknung oder anderer Techniken wie die sogenannte "supercritical fluid technology".

Die Partikel können sich durch ein besonders vorteilhaftes Freisetzungsverhalten auszeichnen, wobei das Freisetzungsverhalten von Zytokinen aus den Partikeln im Wesentlichen durch die Größe und die Zusammensetzung der Partikel sowie die Konzentration und Auswahl der darin eingeschlossenen Zytokine bestimmt wird. Bevorzugt zeichnen sich die Partikel der erfindungsgemäßen Zusammensetzung dadurch aus, dass die Freisetzung der Zytokine aus den Partikeln kontinuierlich über mehrere Tage hinweg erfolgt, insbesondere kann die Freisetzung nach einer ersten Startphase von 24h bis 36h im Wesentlichen linear erfolgen. Besonders bevorzugt zeichnen sich die Partikel der erfindungsgemäßen Zusammensetzung dadurch aus, dass die Freisetzung der Zytokine mit einer Rate erfolgt von nicht mehr als 50 Gew.-% der eingeschlossenen Zytokine pro 24h, ganz besonders bevorzugt von nicht mehr als 50 Gew.-% der eingeschlossenen Zytokine pro 48h.

Die erfindungsgemäße Zusammensetzung kann zusätzlich Fibrinogen enthalten. Die Zugabe von Fibrinogen bewirkt, dass die Zusammensetzung nach Implantation am Ort stabilisiert wird und nicht schnell abgetragen werden kann. Durch den Kontakt mit Blut härtet das Fibrinogen schnell aus und fixiert so die Zusammensetzung an dem gewünschten Ort im Gelenk. Dies ist insbesondere dann wünschenswert, wenn die Zusammensetzung als Gel oder Paste arthroskopisch in die Läsion appliziert wird. Dadurch ist es möglich klein- bis großflächige, lokal begrenzte osteoarthrotische Knorpeldefekte mit der erfindungsgemäßen Zusammensetzung in Form einer Paste oder eines Gels mittels arthroskopischer Techniken minimal-invasiv zu behandeln oder aufzufüllen. Bevorzugt enthält die erfindungsgemäße Zusammensetzung 10 mg/ml bis 300 mg/ml Fibrinogen, besonders bevorzugt 70 mg/ml bis 110 mg/ml, jeweils bezogen auf das Gesamtvolumen (w/v) der Zusammensetzung.

Die Bestandteile der erfindungsgemäßen Zusammensetzung, insbesondere die Hyaluronsäure, und die mit Zytokinen beladenen Partikel, können in einem Lösungsmittel gelöst, emulgiert oder suspendiert vor. Bevorzugt ist das Lösungsmittel ein wässriges Lösungsmittel, wie beispielsweise Wasser. Da die erfindungsgemäße Zusammensetzung sich zur Verwendung als Medikament oder als aktiver Bestandteil eines Medikamentes eignet, ist es besonders vorteilhaft, wenn es sich bei dem Lösungsmittel um eine infundierbare wässrige Lösung handelt, beispielsweise eine isotonische Kochsalzlösung. Die infundierbare wässrige Lösung und insbesondere die isotonische Kochsalzlösung kann gepuffert sein, um den pH-Wert auf einem physiologisch akzeptablen Niveau zu halten bzw. um einen physiologischen pH-Wert herzustellen. Geeignete Puffer sind dem Fachmann bekannt.

Die vorliegende Erfindung bezieht sich auch auf eine pharmazeutische Zusammensetzung enthaltend eine erfindungsgemäße Zusammensetzung und einen oder mehrere pharmazeutisch verträgliche Hilfsstoffe. Der Begriff "Hitfsstoff" wird hierin zur Beschreibung aller anderen Bestandteile außer der erfindungsgemäßen Zusammensetzung verwendet Die Auswahl des oder der Hilfsstoffe richtet sich weitgehend nach der speziellen Art der Verabreichung. Dabei können beispielsweise Hilfsstoffe für die Formulierung eingesetzt werden und/oder Stabilisatoren, Konservierungsstoffe, Hilfsstoffe zur Einstellung der Viskosität, Antioxidantien, Farbstoffe, Bindemittel, Emulgatoren, Feuchthaltemittel, Lösungsmittel, Füllmittel, Salze, Kohlenhydrate und/oder Puffersubstanzen. Bevorzugt handelt es sich bei den Hilfsstoffen um Salze, Kohlenhydrate und/oder Puffersubstanzen, die zur parenteralen Verabreichung, insbesondere zur Implantation in den Körper eines zu behandelnden Individuums, geeignet sind.

In einer besonderen Ausführungsform ist die erfindungsgemäße pharmazeutische Zusammensetzung für die intra-artikuläre Injektion hergerichtet. Dazu kann die Konzentration der Partikel in der pharmazeutischen Zusammensetzung 50 mg/ml und die der Hyaluronsäure 10 mg/ml betragen, jeweils bezogen auf das Gesamtvolumen (w/v) der pharmazeutischen Zusammensetzung. Die pharmazeutische Zusammensetzung liegt bevorzugt als injizierbare wässrige Lösung vor, besonders bevorzugt sind die Bestandteile der erfindungsgemäßen Zusammensetzung in einer isotonischen, ggf. gepufferten Kochsalzlösung gelöst, emulgiert oder suspendiert.

In einer anderen Ausführungsform ist die erfindungsgemäße pharmazeutische Zusammensetzung für die arthroskopische Behandlung hergerichtet. Die pharmazeutische Zusammensetzung liegt hierfür bevorzugt als Gel oder Paste vor. Dazu kann die Konzentration der Partikel in der pharmazeutischen Zusammensetzung 200 mg/ml, die der Hyaluronsäure 20 mg/ml betragen, jeweils bezogen auf das Gesamtvolumen (w/v) der pharmazeutischen Zusammensetzung. Zusätzlich kann die pharmazeutische Zusammensetzung 70 - 110 mg/ml Fibrinogen enthalten, bezogen auf das Gesamtvolumen (w/v) der pharmazeutischen Zusammensetzung.

Die erfindungsgemäße Zusammensetzung bzw. die erfindungsgemäße pharmazeutische Zusammensetzung kann zur Behandlung und/oder Prophylaxe von Knorpeldefekten, beispielsweise von traumatischen Knorpeldefekten oder Osteoarthrose verwendet werden, insbesondere zur Behandlung und/oder Prophylaxe von Osteoarthrose in Gelenken, beispielsweise in Knie-, Sprung-, Schulter- und/oder Hüftgelenken. Bevorzugt wird die erfindungsgemäße Zusammensetzung bzw. die erfindungsgemäße pharmazeutische Zusammensetzung zur Behandlung und/oder Prophylaxe von Knorpeldefekten bei Säugetieren, insbesondere bei Menschen und/oder Nutz- oder Haustieren verwendet.

Die vorliegende Erfindung betrifft auch die erfindungsgemäße Zusammensetzung bzw. die erfindungsgemäße pharmazeutische Zusammensetzung zur Verwendung in der Behandlung und/oder Prophylaxe von Knorpeldefekten, beispielsweise von traumatischen Knorpeldefekten oder Osteoarthrose, insbesondere der Osteoarthrose in Knie-, Sprung-, Schulter- und/oder Hüftgelenken.

Die vorliegende Erfindung bezieht sich auch auf ein Verfahren zur Behandlung und/oder Prophylaxe von Knorpeldefekten, beispielsweise von traumatischen Knorpeldefekten oder Osteoarthrose, wobei einem Individuum, welches einer solchen Therapie bedarf, eine wirksame Dosis der erfindungsgemäßen Zusammensetzung oder der erfindungsgemäßen pharmazeutischen Zusammensetzung verabreicht wird.

Gemäß vorliegender Erfindung wird die Zusammensetzung bzw. die pharmazeutische Zusammensetzung vorzugsweise in einer wirksamen Dosis verabreicht. Eine "wirksame Dosis" ist die Dosis der erfindungsgemäßen Zusammensetzung, die bei Verabreichung an ein Individuum eine messbare therapeutische Wirkung im Hinblick auf die fragliche Erkrankung bewirkt. Bei der vorliegenden Erfindung ist eine wirksame Dosis diejenige Dosis der erfindungsgemäßen Zusammensetzung, die eine therapeutische Wirkung im Hinblick auf die zu behandelnden Knorpeldefekte hervorbringt. Vorzugsweise wird die erfindungsgemäße Zusammensetzung in einer Dosis von 0,1 bis 10 ml pro zu behandelndem Gelenk verabreicht, bevorzugt von 1 ml bis 3 ml pro zu behandelndem Gelenk. Um einen besonders ausgeprägten Behandlungserfolg zu erzielen, wird das zu behandelnde Gelenk mit der erfindungsgemäßen Zusammensetzung mehr als einmal behandelt. Bevorzugt wird die Behandlung eines betroffenen Gelenks ein- bis dreimal wiederholt, so dass es insgesamt zu zwei bis vier Behandlungen pro Gelenk und Behandlungszyklus kommen kann. Dabei finden die Wiederholungen bevorzugt in einem Abstand von jeweils 4 bis 9 Tagen statt, besonders bevorzugt in einem Abstand von jeweils 7 Tagen.

Die vorliegende Erfindung bezieht sich auch auf die Verwendung einer erfindungsgemäßen Zusammensetzung bzw. einer erfindungsgemäßen pharmazeutischen Zusammensetzung zur Herstellung eines Medikaments zur Behandlung und/oder Prophylaxe von Knorpeldefekten, beispielsweise von traumatischen Knorpeldefekten oder Osteoarthrose, insbesondere von Osteoarthrose von Knie-, Sprung-, Schulter- und/oder Hüftgelenken, wobei das Medikament zur Verabreichung von 0,1 ml bis 10 ml, bevorzugt von 1 ml bis 3 ml, der Zusammensetzung pro zu behandelndem Gelenk und ggf. zur ein- bis dreimaligen Wiederholung der Behandlung im Abstand von jeweils 4 bis 9 Tagen hergerichtet ist.

Die Behandlung eines von Osteoarthrose betroffenen Gelenks mit einer erfindungsgemäßen Zusammensetzung bzw. mit einer erfindungsgemäßen pharmazeutischen Zusammensetzung kann insbesondere mit anderen Therapieformen zur Behandlung von Osteoarthrose kombiniert werden. Insbesondere die Darreichungsform der erfindungsgemäßen pharmazeutischen Zusammensetzung als Paste oder Gel für die arthroskopische Verabreichung kann nach einer arthroskopischen Behandlung umfassend eine Mikrofrakturierung und/oder Pridie-Bohrungen verabreicht werden.

In einer weiteren Ausführungsform bezieht sich die vorliegende Erfindung auf eine vorgefüllte Spritze, die mit einer injizierbaren Suspension gefüllt ist, die eine erfindungsgemäße Zusammensetzung bzw. eine erfindungsgemäße pharmazeutische Zusammensetzung enthält oder daraus besteht. Diese vorgefüllte Spritze kann zur Behandlung und/oder Prophylaxe von Knorpeldefekten, beispielsweise von traumatischen Knorpeldefekten oder Osteoarthrose, insbesondere von Osteoarthrose von Knie-, Sprung-, Schulter- und/oder Hüftgelenken, verwendet werden.

Im Folgenden wird die Erfindung anhand von Ausführungsbeispielen näher verdeutlicht.

### Figuren:

Es zeigen
- Figur 1.:: die Größenverteilung und Morphologie der hergestellten PLGA-Chemokin Partikel, **(A)** Größenverteilungskurve, **(B), (C)** repräsentative rasterelektronenmikroskopische Bilder;
- Figur 2:: die Degradation der PLGA-Chemokin Partikel in wässriger Umgebung: **(A)** Massenverlust der Partikel über die Zeit, **(B)** morphologische Veränderungen der Partikel anhand rasterelektronenmikroskopischer Untersuchungen;
- Figur 3:: einen Phagozytose-Assay der Chemokin-beladenen Partikel;
- Figur 4:: kumulative Freisetzungskinetiken aus den PLGA Partikeln über mehrere Wochen für die Chemokine CXCL12 **(A)** und CCL25 **(B);**
- Figur 5:: die Expressionen verschiedener knorpeltypischer Gene sind hoch reguliert bei der chondrogenen Differenzierung von mesenchymalen Stamm- und Progenitorzellen mit Hyaluronsäure, d = day / Tag; **(A)** Kollagen Typ II, **(B)** cartilage link protein, **(C)** cartilage oligomeric matrix protein, **(D)** aggrecan;
- Figur 6:: einen Chemotaxis-Assay mit mesenchymalen Stamm- und Progenitorzellen unter Stimulation mit verschiedenen Konzentrationen CCL25 **(A)** und CXCL12 **(B);**
- Figur 7:: einen Chemotaxis-Assay: Hyaluronsäure stimuliert die Migration von mesenchymalen Stamm- und Progenitorzellen; und
- Figur 8:: einen Chemotaxis-Assay: Die Kombination von HA (HS) und Chemokinen CCL25 bzw. CXCL12 stimuliert synergistisch die Wanderung von mesenchymalen Stamm- und Progenitorzellen; HS = HA, Hyaluronsäure; **(A)** Kombination HA mit CCL25, **(B)** Kombination HA mit CXCL12.

### Beispiele:

### Beispiel 1: Herstellung von Partikeln mit darin eingeschlossenen Zytokinen

Für die Verkapselung des Chemokins CXCL12 wurde die Technik der Wasser-in-Öl-in-Wasser (w/o/w) Doppelemulsion mit anschließender Lösungsmittelverdunstung angewendet. Die Herstellung der w/o/w-Emulsion wurde in einem Dreischrittverfahren durchgeführt.

Im ersten Schritt erfolgte das Verteilen der chemokinhaltigen wässrigen Phase (w₁) in der PLGA-Ölphase (o). Das führte zum Entstehen der Primäremulsion (w₁/o). Im zweiten Schritt wurde die Primäremulsion in einer äußeren wässrigen Polyvinylalkohol (PVA)-Phase (w₂) dispergiert. Dabei wurde eine Sekundäremulsion (w₁/o/w₂) erzeugt, die anschließend in einer zweiten äußeren wässrigen Phase verdünnt wurde.

Für die Herstellung der w₁-Phase wurden 10 µg Chemokin (CXCL12) in 100 µl Aqua dest. gelöst, um eine Ausgangskonzentration von 100 ng/µl zu erhalten. In den weiteren unterschiedlichen Ansätzen wurden 50 µl von der Ausgangskonzentration in (1) 450 µl PBS, (2) 450 µl 10 % HSA oder (3) in 450 µl Heparinlösung verdünnt, um die im Releaseansatz verwendete Endkonzentration von 10 ng/µl Chemokinlösung (w₁) zu erhalten.

Zur Erzeugung von 1 % PVA und von 0,5 % PVA/0,45 M NaCl als äußere wässrige Phasen (w₂-Phasen) wurden entsprechend 1g PVA in 100 ml und 5 g PVA in 1000 ml Aqua dest. unter Rühren und Wärmezufuhr gelöst. Weiterhin wurden zu der 0,5 % PVA drei Tabletten NaCl (entspricht 0,45 M) zugegeben und auch unter Rühren gelöst. Für die Herstellung der organischen Polymerphase (o) wurden 100 mg PLGA in 1 ml Methylenchlorid gelöst.

Die Primäremulsion (w₁/o) wurde durch Zugabe der ersten wässrigen CXCL12 Phase (w₁; 10 µl der Chemokinlösung (10 ng/µl), entspricht 100 ng) zu der PLGA-Ölphase (o; 100 mg Polymer in 1 ml Methylenchlorid) erzeugt. Dabei wurde die Emulsion 3 Minuten lang gevortext Für die Erzeugung der Doppelemulsion (w₁/o/w₂) wurde die Primäremulsion in 2 ml einer äußeren wässrigen 1 %igen PVA-Phase (w₂) durch 3-minütiges Vortexen dispergiert. Anschließend wurde die Doppelemulsion in 20 ml 0,5 % PVA/0,45 M NaCl in Bechergläser überführt und bei 60 g auf einem Magnetrührer durchmischt. Während des 4-stündigen Rührens verdampfte die organische Phase und die CXCL12-Partikel härteten aus. Zum Schluss wurden die Partikel dreimal mit 20 ml Aqua dest. gewaschen und anschließend über Nacht in einem Gefriertrockner getrocknet.

### Beispiel 2: Die hergestellten PLGA-Chemokin Partikel haben eine enge Größenverteilung

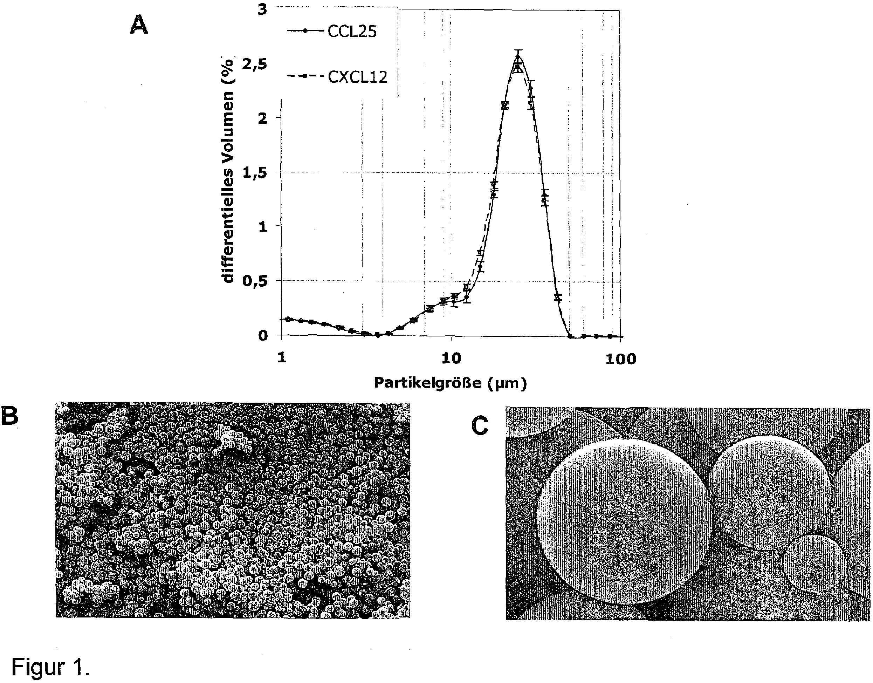

Chemokin-verkapselnde PLGA Partikel wurden nach der w/o/w Doppelemulsion mit Lösemittelverdampfung hergestellt und anschließend vergleichend für CXCL12- und CCL25-verkapselnde Partikel charakterisiert. Die Partikel besitzen eine enge und reproduzierbare Größenverteilung, nur 10% der Partikel sind kleiner als 6 µm und kein Partikel ist größer als 50 µm. Der durchschnittliche mittlere Partikeldurchmesser beträgt 21 µm (siehe Figur 1).

Die PLGA-Chemokin Partikel weisen eine runde Morphologie mit einer hohen strukturellen Integrität auf. Die Oberfläche der Partikel ist glatt, unversehrt und ohne sichtbare Poren oder Aggregationen. Die Partikel besitzen eine negative Oberflächenladung (zeta Potential = - 26,64 mV ± 0,72 mV).

### Beispiel 3: PLGA-Chemokin Partikel degradieren in wässrigen Lösungen

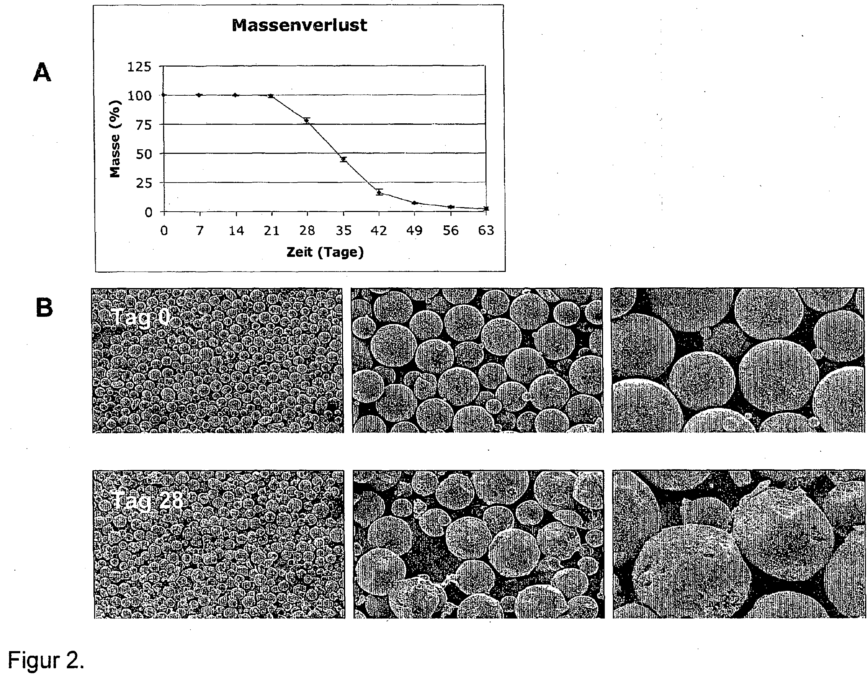

Die hergestellten PLGA-Chemokin Partikel sind biodegradierbar, PLGA degradiert durch hydrolytische Spaltung der Esterbindungen unter physiologischen Bedingungen in wässriger Umgebung. Die Degradationsendprodukte Milch- und Glycolsäure sind in den auftretenden Konzentrationen nicht toxisch und werden vollständig verstoffwechselt.
Die Degradation der PLGA-Chemokin Partikel wurde anhand des Massenverlustes (siehe Figur 2A) und morphologischer Veränderung im Rasterelektronenmikroskop (siehe Figur 2B) untersucht.

In wässrigem Milieu degradieren die hergestellten PLGA-Chemokin Partikel. Der Massenverlust der Partikel beginnt nach drei Wochen Inkubation in wässriger Lösung, nach 7 Wochen ist bereits ein Massenverlust von 84 % erfolgt und nach 9 Wochen ist nur noch 2 % der Masse vorhanden. Auch morphologisch lässt sich eine Veränderung der Partikel durch die Degradation beobachten: Während die Partikel zu Beginn noch eine runde, glatte und porenlose Oberfläche aufweisen, zeigt sich nach 28 Tagen Degradation bereits eine vermehrte Porenbildung an der Partikeloberfläche und ein Zerfall der Partikel.

### Beispiel 4: PLGA-Chemokin Partikel werden von Leukozyten nicht phagozytiert

Die Phagozytose der injizierten Chemokin-beladenen Partikel durch Leukozyten im Körper muss ausgeschlossen werden, da dies sowohl zu einer Entzündungsreaktion als auch zu einer vorzeitigen Beseitigung des Partikel-Wirkstoff-Freisetzungssystems führen kann.

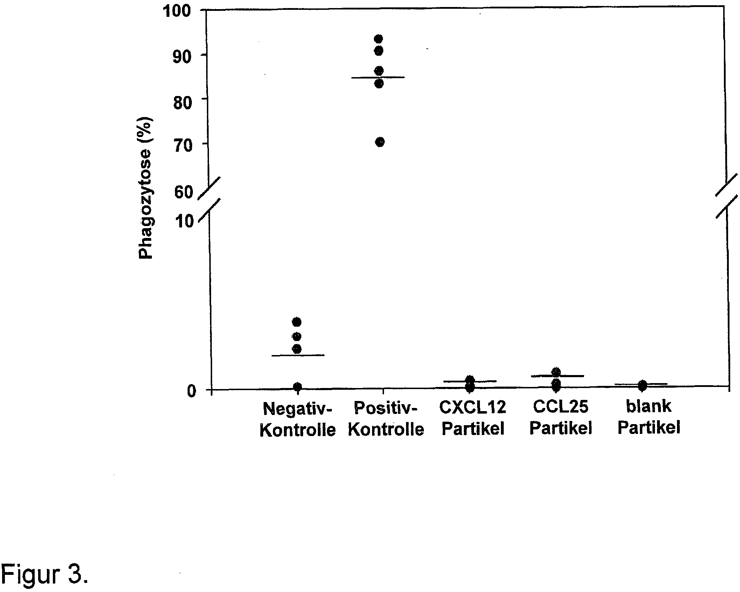

In einem Phagozytose-Assay wurden die PLGA-Chemokin Partikel mit heparinisiertem Vollblut bei 37 °C inkubiert und der Anteil der Phagozytose-aktiven Leukozyten bestimmt. Während die Positivkontrolle (opsonisierte Bakterien bei 37 °C) zu ca. 85 % von den Leukozyten phagozytiert wird, werden die Negativkontrolle (opsonisierte Bakterien bei 4 °C) und die Chemokin-Partikel für CXCL12 und CCL25 bzw. "leere" blank-Partikel (bei 37 °C) kaum phagozytiert (siehe Figur 3). Vermutlich sind die Partikel mit einem durchschnittlichen mittleren Partikeldurchmesser von 21 µm für eine phagozytotische Aufnahme durch Leukozyten zu groß.

### Beispiel 5: Lineare Chemokin-Freisetzungsprofile aus PLGA-Partikeln

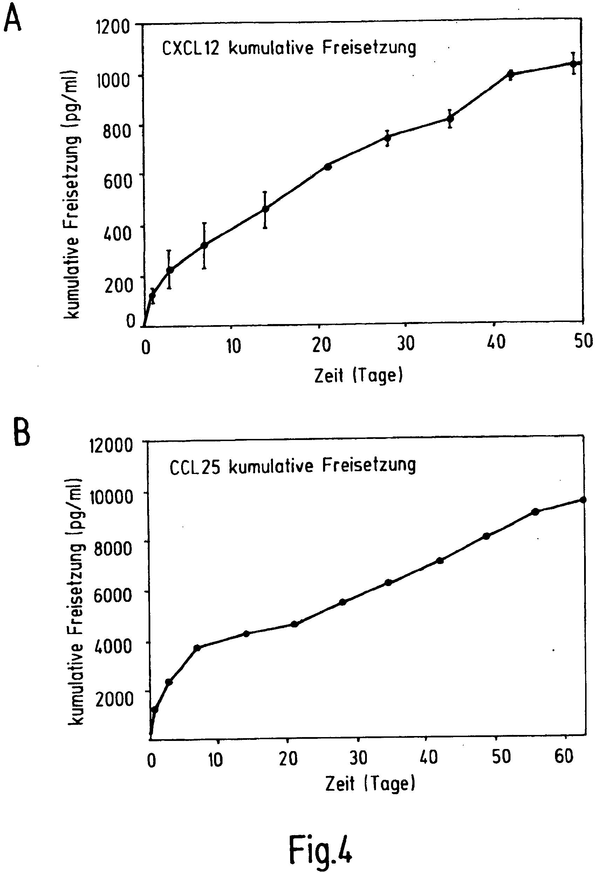

Die Freisetzung von verkapselten Zytokinen aus PLGA Partikeln erfolgt in wässriger Lösung durch Diffusion des Wirkstoffes aus den Partikeln und infolge Degradation der Polymerketten mit einhergehender Verkürzung der Polymerketten und Vergrößerung der Poren an der Oberfläche der Partikel. Die kumulative Freisetzungskinetik aus den Polymerpartikeln wurde für die Chemokine CXCL12 und CCL25 mittels *enzyme-linked immunosorbant assay* (ELISA) untersucht. Die Ergebnisse sind in Figur 4 dargestellt.

Die Freisetzung beider Chemokine aus den PLGA-Partikeln weist ein insgesamt nahezu lineares Profil auf. Es zeigt sich kaum ein burst Release und anschließend eine nahezu lineare Freisetzung der Chemokine aus den PLGA Partikeln für mehrere Wochen. Nach 50 bis 60 Tagen sind die Partikel nahezu vollständig degradiert und setzen kaum noch Chemokin frei.

### Beispiel 6: Hyaluronsäure wirkt positiv auf die Knorpelbildung

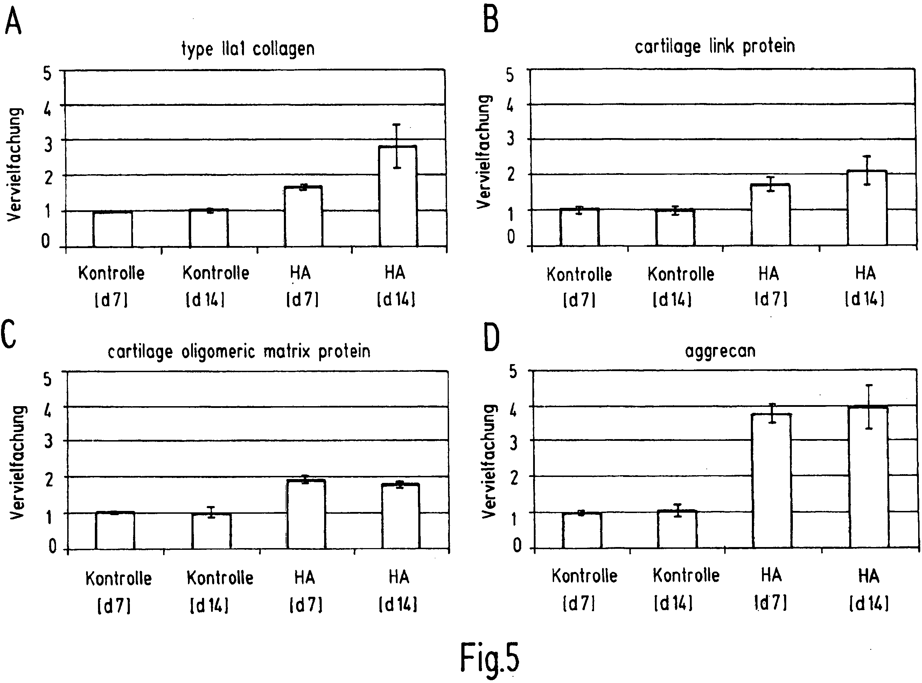

Die chondrogene Differenzierung von mesenchymalen Stamm- und Progenitorzellen (MSCs) wird auf Genexpressionsebene durch HA stimuliert. Bei der chondrogenen Differenzierung mit HA sind verschiedene knorpeltypische Markergene (Kollagen Typ 2, cartilage link protein, cartilage oligomeric matrix protein, aggrecan) in ihrer Expression hoch reguliert (siehe Figur 5).

Diese chondro-stimulierende Wirkung von HA auf mesenchymale Stamm- und Progenitorzellen konnte auch auf histologischer Ebene bestätigt werden.

Nach der 26-tägigen Chondrogenese von mesenchymalen Stamm- und Progenitorzellen mit HA zeigte sich die knorpeltypische Anfärbung von Proteoglykanen nach der Alzianblau Färbung und von Kollagen Typ 2 nach immunhistochemischer Färbung.

### Beispiel 7: Mesenchymale Stamm- und Progenitorzellen können durch Chemokine angelockt werden (Chemotaxis)

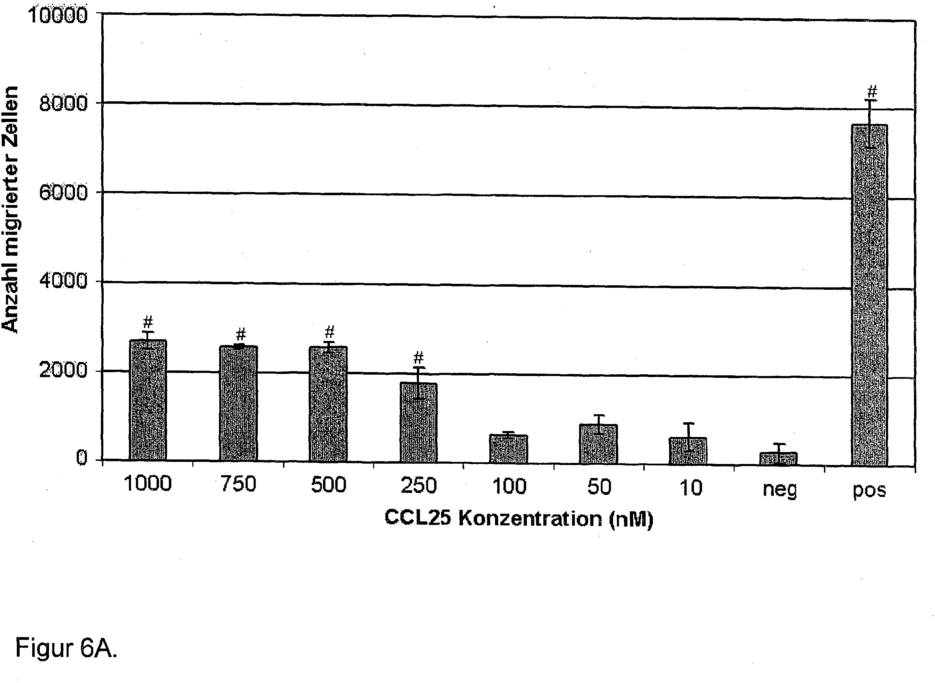

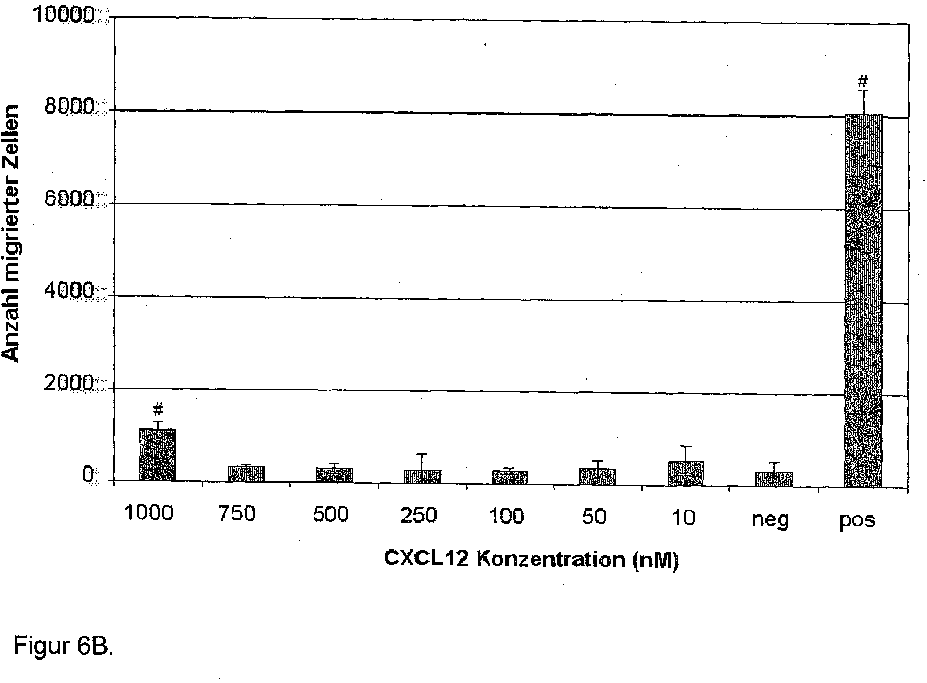

Chemokine sind chemotaktische Zytokine, die eine gerichtete Migration von Zellen auslösen. In einem Migrationsassay wurde die chemotaktische Wirkung der beiden Chemokine CCL25 und CXCL12 auf mesenchymale Stamm- und Progenitorzellen (MSCs) untersucht. Dabei werden die MSCs in Diätmedium (beinhaltet neben dem Standardmedium 0,1 % fötales Kälberserum, ohne zusätzliche Chemokine) aufgenommen und auf eine Poren-durchsetzte Membran gebracht. Unterhalb der Membran befindet sich das Chemokin in verschiedenen Konzentrationen (10 nM - 1000 nM), Diätmedium (Negativkontrolle) bzw. Expansionsmedium (Positivkontrolle, beinhaltet neben dem Standardmedium 10 % fötales Kälberserum, ohne zusätzliche Chemokine). Nach einer Zellmigration können die gewanderten Zellen an der Membranunterseite detektiert und ausgezählt werden. Anhand der Positivkontrolle wird die Gesamtzahl der wanderungsfähigen Zellen bestimmt, die Negativkontrolle dient zur Bestimmung der gewanderten Zellen ohne Stimulation mit Chemokinen.
Es konnte gezeigt werden, dass beide Chemokine CXCL12 (siehe Figur 6B) und CCL25 (siehe Figur 6A) konzentrationsabhängig die Wanderung von MSCs stimulieren.

Das Chemokin CCL25 induziert schon ab einer Konzentration von 250 nM signifikant die Migration der MSCs im Vergleich zu der Negativkontrolle. Nahezu ein Viertel der Gesamtwanderungsfähigen Zellen kann ab einer Konzentration von 250 nM CCL25 rekrutiert werden. Das Chemokin CXCL12 rekrutierte dagegen erst bei einer Konzentration von 1000 nM signifikant MSCs. Im Durchschnitt konnte hier 1/8 der gesamten wanderungsfähigen Zellen angelockt werden.

### Beispiel 8: Mesenchymale Stamm- und Progenitorzellen können durch Hyaluronsäure angelockt werden (Chemotaxis)

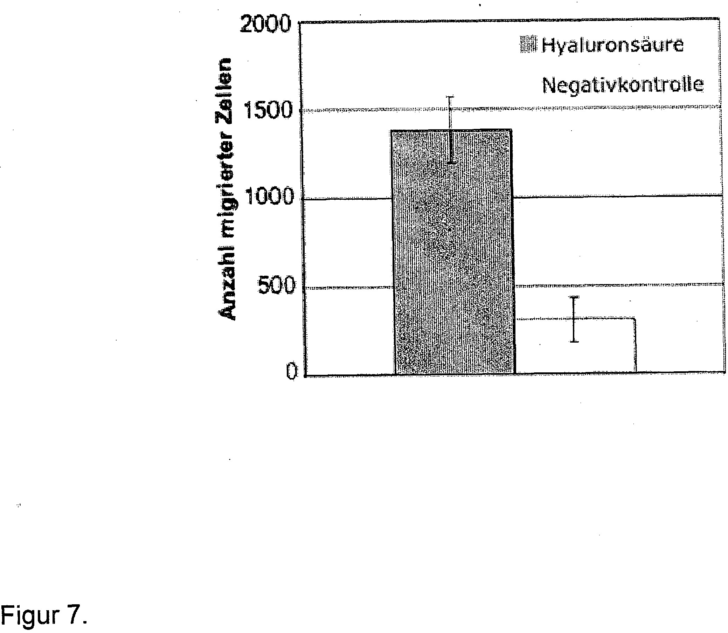

Neben der chondro-induktiven Wirkung von HA auf MSCs (Beispiel 6) konnte außerdem gezeigt werden, dass HA auch als Chemoattraktant auf MSCs wirkt. In einem Chemotaxis-Assay konnte gezeigt werden, dass HA bei einer Konzentration von 5 mg/mL die Migration von MSCs deutlich stimuliert im Vergleich zur Negativkontrolle ohne HA (siehe Figur 7).

### Beispiel 9: Die Kombination von Hyaluronsäure und Chemokin stimuliert synergistisch die Migration von mesenchymalen Stamm- und Progenitorzellen

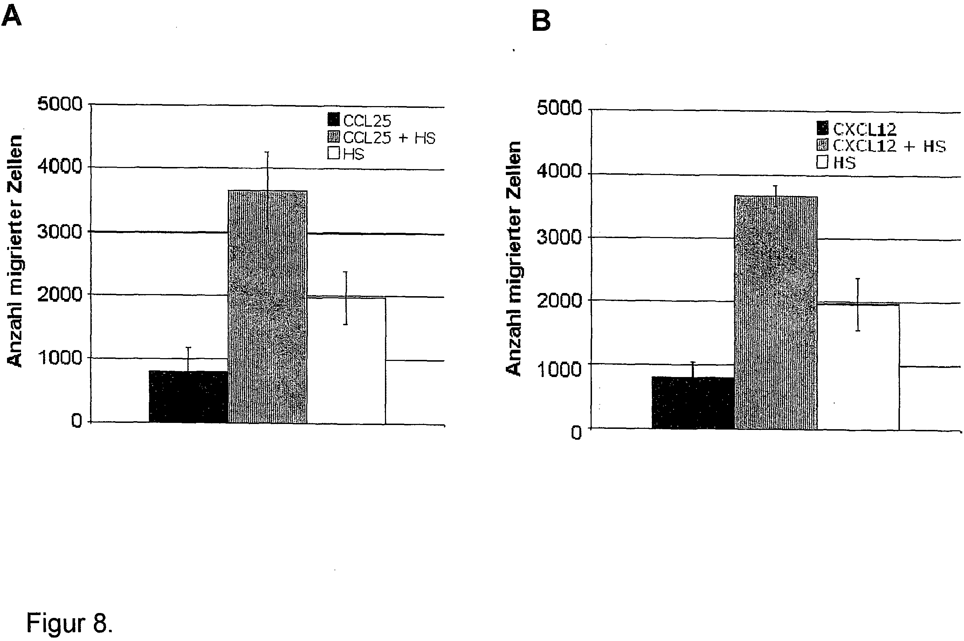

Wird HA und Chemokin in Kombination verabreicht, so ist der chemotaktische Effekt auf mesenchymale Stamm- und Progenitorzellen größer, als die Addition beider Einzeleffekte (siehe Figur 8).

Die Chemokine CCL25 und CXCL12 rekrutieren in einer Konzentration von 100 nM ca. 800 mesenchymale Stamm- und Progenitorzellen. Hyaluronsäure lockt bei einer Konzentration von 2 mg/mL ca. 2000 dieser Zellen an. Wird beides zu gleichen Konzentrationen zusammen verabreicht (CCL25/HA bzw. CXC12/HA), so werden im Durchschnitt 3700 MSCs rekrutiert. Die kombinierte Verabreichung der Chemokine und HA hat demnach einen synergistischen Effekt auf die Migration von mesenchymalen Stamm- und Progenitorzellen.

## Patentansprüche

1. Zusammensetzung enthaltend oder bestehend aus:
| | |
|---|---|
| 2 - 50 mg/ml | Hyaluronsäure, |
| 0,1 - 500 mg/ml | biodegradierbare Partikeln mit einem durchschnittlichen mittleren Partikeldurchmesser von 1 nm - 500 µm, |
| 1 pg/ml- 10 µg/ml | Zytokine, wobei es sich bei den Zytokinen um CXCL10, CXCL12, CCL25 oder XCL1 handelt oder um eine Mischung enthaltend eines oder mehrere davon, |
wobei sich die Konzentrationsangaben jeweils auf das Gesamtvolumen (w/v) der Zusammensetzung beziehen und wobei die Zytokine in den biodegradierbaren Partikeln eingeschlossen sind.

2. Zusammensetzung nach Anspruch 1, **dadurch gekennzeichnet, dass** die Zusammensetzung 5- 30 mg/ml Hyaluronsäure enthält.

3. Zusammensetzung nach einem der vorhergehenden Ansprüche, **dadurch gekennzeichnet, dass** die Zusammensetzung 1 - 300 mg/ml Partikel enthält.

4. Zusammensetzung nach einem der vorhergehenden Ansprüche, **dadurch gekennzeichnet, dass** die Partikel eine durchschnittliche mittlere Partikelgröße von 10 nm - 200 µm aufweisen.

5. Zusammensetzung nach einem der vorhergehenden Ansprüche, **dadurch gekennzeichnet, dass** die Zusammensetzung die in den Partikeln eingeschlossenen Zytokine in einer Konzentration von 10 pg/ml - 1 µg/ml enthält.

6. Zusammensetzung nach einem der vorhergehenden Ansprüche, **dadurch gekennzeichnet, dass** die Partikel ein biokompatibles, biodegradierbares Polymer enthalten oder daraus bestehen.

7. Zusammensetzung nach Anspruch 6, **dadurch gekennzeichnet, dass** das biocompatible, biodegradierbare Polymer ausgewält ist aus Polymeren oder Co-Polymeren, die ein PLA- und/oder ein PGA-Monomer enthalten, PLGA, PLGA-REG, Polycaprolactone, Polycarbonate, Polyamide, Polyanhydride, PEG, Chitosane, Dextrane, Cyclodextrine und/oder Fibrinogen.

8. Zusammensetzung nach einem der vorhergehenden Ansprüche, **dadurch gekennzeichnet, dass** die Zusammensetzung zusätzlich ein wässriges Lösungsmittel enthält, beispielsweise Wasser oder eine infundierbare wässrige Lösung, insbesondere isotonische Kochsalzlösung, besonders bevorzugt gepufferte isotonische Kochsalzlösung.

9. Zusammensetzung nach einem der vorhergehenden Ansprüche, **dadurch gekennzeichnet, dass** die Zusammensetzung zusätzlich 10 - 300 mg/ml Fibrinogen enthält.

10. Pharmazeutische Zusammensetzung enthaltend eine Zusammensetzung nach einem der Ansprüche 1 bis 9 und mindestens einen pharmazeutisch verträglichen Hilfsstoff. .

11. Pharmazeutische Zusammensetzung nach Anspruch 10 für die intra-artikuläre Injektion, **dadurch gekennzeichnet, dass** die Konzentration der Partikel in der pharmazeutischen Zusammensetzung 50 mg/ml und die der Hyaluronsäure 10 mg/ml beträgt.

12. Pharmazeutische Zusammensetzung nach Anspruch 10 für die arthroskopische Behandlung, **dadurch gekennzeichnet, dass** die Konzentration der Partikel in der pharmazeutischen Zusammensetzung 200 mg/ml, die der Hyaluronsäure 20 mg/ml beträgt und die pharmazeutische Zusammensetzung 70 - 110 mg/ml Fibrinogen enthält.

13. Zusammensetzung nach einem der Ansprüche 1 bis 9 oder einer pharmazeutischen Zusammensetzung nach einem der Ansprüche 10 bis 12 zur Verwendung in der Behandlung und/oder Prophylaxe von Knorpeldefekten.

14. Verwendung einer pharmazeutischen Zusammensetzung nach einem der Ansprüche 10 bis 12 zur Herstellung eines Medikaments zur Behandlung und/oder Prophylaxe von Knorpeldefekten, wobei das Medikament zur Verabreichung von 0,1 bis 10 ml der pharmazeutischen Zusammensetzung pro zu behandelnde Gelenk und ggf. zur ein-bis dreimaligen Wiederholung der Behandlung im Abstand von jeweils 4 bis 9 Tagen hergerichtet ist.

## Claims

1. A composition containing or consisting of:
| | |
|---|---|
| 2 - 50 mg/ml | hyaluronic acid, |
| 0,1 - 500 mg/ml | biodegradable particles with an average mean particle diameter of 1 nm - 500 µm, |
| 1 pg/ml - 10 µg/ml | cytokines, wherein the cytokines are CXCL10, CXCL12, CCL25 or XCL1 or a mixture containing one or several thereof, |
wherein each of the aforesaid concentrations relates to the total volume (w/v) of the composition, and wherein the cytokines are enclosed in the biodegradable particles.

2. The composition according to claim 1, **characterized in that** the composition contains 5 - 30 mg/ml of hyaluronic acid.

3. The composition according to any one of the preceding claims, **characterized in that** the composition contains 1 - 300 mg/ml of the particles.

4. The composition according to any one of the preceding claims, **characterized in that** the particles have an average mean particle size of 10 nm - 200 µm.

5. The composition according to any one of the preceding claims, **characterized in that** the cytokines enclosed in the particles are contained in the composition in a concentration of 10 pg/ml - 1 µg/ml.

6. The composition according to any one of the preceding claims, **characterized in that** the particles contain or consist of a biocompatible, biodegradable polymer.

7. The composition according to claim 6, **characterized in that** the biocompatible, biodegradable polymer is selected from polymers or co-polymers which contain a PLA monomer and/or a PGA monomer, PLGA, PLGA-PEG, polycaprolactones, polycarbonates, polyamides, polyanhydrides, PEG, chitosans, dextrans, cyclodextrins and/or fibrinogen.

8. The composition according to any one of the preceding claims, **characterized in that** the composition additionally contains an aqueous solvent, for example water or an infusible aqueous solution, in particular isotonic saline solution, more preferably buffered isotonic saline solution.

9. The composition according to any one of the preceding claims, **characterized in that** the composition additionally contains 10 - 300 mg/ml of fibrinogen.

10. A pharmaceutical composition containing a composition according to any one of claims 1 to 9 and at least one pharmaceutically tolerable excipient.

11. The pharmaceutical composition according to claim 10 for intra-articular injection, **characterized in that**, in the pharmaceutical composition, the concentration of the particles is 50 mg/ml and the concentration of hyaluronic acid is 10 mg/ml.

12. The pharmaceutical composition according to claim 10 for arthroscopic treatment, **characterized in that**, in the pharmaceutical composition, the concentration of the particles is 200 mg/ml, the concentration of hyaluronic acid is 20 mg/ml, and the pharmaceutical composition contains 70 - 110 mg/ml of fibrinogen.

13. The composition according to any one of claims 1 to 9 or a pharmaceutical composition according to any one of claims 10 to 12 for use in the treatment and/or prevention of cartilage defects.

14. Use of a pharmaceutical composition according to any one of claims 10 to 12 for the production of a medicine for the treatment and/or prevention of cartilage defects, which medicine is adapted for the administration of 0.1 to 10 ml of the pharmaceutical composition per joint to be treated and, as the case may be, for repetition of the treatment one to three times at intervals of 4 to 9 days.

## Revendications

1. Composition, contenant ou composée de:
| | |
|---|---|
| 2 - 50 mg/ml | d'acide hyaluronique, |
| 0,1 - 500 mg/ml | de particules biodégradables ayant un diamètre moyen de particules de 1 nm - 500 µm, |
| 1 pg/ml - 10 µg/ml | de cytokines, s'agissant en matière de cytokines de CXCL10, CXCL12, CCL25 ou XCL1 ou d'un mélange contenant une ou plusieurs de celles-ci, |
les indications de concentration se rapportant respectivement au volume total (w/v) de la composition, et les cytokines étant incluses dans les particules biodégradables.

2. Composition selon la revendication 1, **caractérisée en ce que** la composition contient 5 - 30 mg/ml d'acide hyaluronique.

3. Composition selon une des revendications précédentes, **caractérisée en ce que** la composition contient 1 - 300 mg/ml de particules.

4. Composition selon une des revendications précédentes, **caractérisée en ce que** les particules présentent une taille de particules moyenne de 10 nm - 200 µm.

5. Composition selon une des revendications précédentes, **caractérisée en ce que** la composition contient les cytokines incluses dans les particules à raison d'une concentration de 10 pg/ml - 1 µg/ml.

6. Composition selon une des revendications précédentes, **caractérisée en ce que** les particules contiennent un polymère biocompatible biodégradable ou se composent de ce polymère.

7. Composition selon la revendication 6, **caractérisée en ce que** le polymère biocompatible biodégradable est sélectionné parmi des polymères ou copolymères qui contiennent un monomère PLA et/ou PGA, PLGA, PLGA-PEG, des polycaprolactones, des polycarbonates, des polyamides, des polyanhydrides, PEG, des chitosanes, des dextranes, des cyclodextrines et/ou du fibrinogène.

8. Composition selon une des revendications précédentes, **caractérisée en ce que** la composition contient en plus un solvant aqueux, par exemple de l'eau, ou une solution aqueuse pouvant infuser, en particulier une solution de sel de cuisine isotonique, de façon particulièrement préférée une solution de sel de cuisine isotonique tamponnée.

9. Composition selon une des revendications précédentes, **caractérisée en ce que** la composition contient en plus 10 - 300 mg/ml de fibrinogène.

10. Composition pharmaceutique contenant une composition selon une des revendications 1 à 9 et au moins un adjuvant compatible au plan pharmaceutique.

11. Composition pharmaceutique selon la revendication 10 pour l'injection intra-articulaire, **caractérisée en ce que** la concentration des particules dans la composition pharmaceutique est égale à 50 mg/ml et celle de l'acide hyaluronique est égale à 10 mg/ml.

12. Composition pharmaceutique selon la revendication 10 pour le traitement arthroscopique, **caractérisée en ce que** la concentration des particules dans la composition pharmaceutique est égale à 200 mg/ml, celle de l'acide hyaluronique est égale à 20 mg/ml et **en ce que** la composition pharmaceutique contient 70 - 110 mg/ml de fibrinogène.

13. Composition pharmaceutique selon une des revendications 1 à 9 ou composition pharmaceutique selon une des revendications 10 à 12 pour l'utilisation dans le traitement et/ou dans la prophylaxie de lésions du cartilage.

14. Utilisation d'une composition pharmaceutique selon une des revendications 10 à 12 pour la fabrication d'un médicament destiné au traitement et/ou à la prophylaxie de lésions du cartilage, le médicament étant préparé pour l'administration de 0,1 à 10 ml de la composition pharmaceutique par articulation à traiter et éventuellement pour une à trois répétitions du traitement à intervalle de respectivement 4 à 9 jours.
